# FASCICULE DE BREVET EUROPEEN

(11) **EP 2 449 093 B1**
(45) Date de publication et mention de la délivrance du brevet: **07.09.2016**
(21) Numéro de dépôt: 10742174.5
(22) Date de dépôt: 01.07.2010
(51) Int. Cl.: C12N 5/071, A61L 27/60, A61L 27/36, A61L 27/38

(54) **EQUIVALENT TISSULAIRE NON RETRACTE, EQUIVALENT DE PEAU COMPORTANT UN TEL EQUIVALENT TISSULAIRE NON RETRACTE ET PROCEDES POUR LA REALISATION D'UN TEL EQUIVALENT TISSULAIRE NON RETRACTE ET D'UN TEL EQUIVALENT DE PEAU**
UNGESCHRUMPFTES GEWEBEÄQUIVALENT, HAUTÄQUIVALENT MIT DERARTIGEM UNGESCHRUMPFTEN EWEBEÄQUIVALENT UND VERFAHREN ZUR HERSTELLUNG EINES DERARTIGEN UNGESCHRUMPFTEN GEWEBEÄQUIVALENTS UND HAUTÄQUIVALENTS
UNSHRUNKEN TISSUE EQUIVALENT, SKIN EQUIVALENT COMPRISING SUCH AN UNSHRUNKEN TISSUE EQUIVALENT, AND METHODS FOR PRODUCING SUCH AN UNSHRUNKEN TISSUE EQUIVALENT AND SUCH A SKIN EQUIVALENT

(30) Priorité: 01.07.2009 FR 0903215; 14.12.2009 FR 0958919
(43) Date de publication de la demande: 09.05.2012
(73) Titulaire: Université de Franche-Comté, 25030 Besançon Cedex (FR); Centre Hospitalier Universitaire de Besançon, 25030 Besançon Cedex (FR)
(72) Inventeur: HUMBERT, Philippe, F-25290 Ornans (FR); BINDA, Delphine, F-25870 Auxon Dessous (FR); VIENNET-STEINER, Céline, F-25290 Ornans (FR); ROBIN, Sophie, F-25480 Pirey (FR); TAUZIN, Hélène, F-25000 Besancon (FR); ROLIN, Gwenaël, F-25000 Besancon (FR); PAZART, Lionel, F-25000 Besancon (FR)
(74) Mandataire: Rhein, Alain
(86) Numéro de dépôt international: PCT/FR2010/051381
(87) Numéro de publication internationale: WO 2011/001116

(56) Documents cités:
- WO-A1-2007/052980
- WO-A2-02/083160
- US-A1- 2005 079 604
- GENTILHOMME E ET AL: "Culture of dermal equivalent submitted to tension" TRAVAUX SCIENTIFIQUES DES CHERCHEURS DU SERVICE DE SANTE DES ARMEES, vol. 0, no. 15, 1994, pages 149-150, XP009128409 ISSN: 0243-7473
- CHAPUIS J F ET AL: "A new technique to study the mechanical properties of collagen lattices" JOURNAL OF BIOMECHANICS, PERGAMON PRESS, NEW YORK, NY, US, vol. 25, no. 1, 1 janvier 1992 (1992-01-01), pages 115-117,119, XP022873208 ISSN: 0021-9290 [extrait le 1992-01-01]
- LAFRANCE H ET AL: "Mechanical properties of human skin equivalents submitted to cyclic tensile forces" SKIN RESEARCH AND TECHNOLOGY 199811 DK, vol. 4, no. 4, novembre 1998 (1998-11), pages 228-236, XP009128407 ISSN: 0909-752X
- KHADEMHOSSEINI A ET AL: "Microscale technologies for tissue engineering and biology" PROCEEDINGS OF THE NATIONAL ACADEMY OF SCIENCES OF THE UNITED STATES OF AMERICA 20060221 US, vol. 103, no. 8, 21 février 2006 (2006-02-21), pages 2480-2487, XP002564980 ISSN: 0027-8424
- PETRULYTE SALVINIJA: "Advanced textile materials and biopolymers in wound management." DANISH MEDICAL BULLETIN FEB 2008 LNKD- PUBMED:18321446, vol. 55, no. 1, février 2008 (2008-02), pages 72-77, XP009138164 ISSN: 1603-9629
- ROLIN G ET AL: "Assessment of tensile strength beneficial effects in an in vitro model of cutaneous wound healing" JOURNAL OF INVESTIGATIVE DERMATOLOGY, vol. 129, no. Suppl. 2, septembre 2009 (2009-09), page S81, XP009128388 & 39TH ANNUAL MEETING OF THE EUROPEAN-SOCIETY-FOR-DERMATOLOGICAL-RESEA RCH; BUDAPEST, HUNGARY; SEPTEMBER 09 -12, 2009 ISSN: 0022-202X

## Description

La présente invention concerne un procédé pour la réalisation d'un équivalent de peau comportant, d'une part, au moins un équivalent de derme constitué par un équivalent tissulaire non rétracté et, d'autre part, au moins un équivalent d'épiderme.

Cette invention a, également, trait à un équivalent de peau notamment obtenu par la mise en oeuvre-de ce procédé.

Ainsi, la présente invention concerne le domaine de la fabrication de l'équivalent d'au moins un tissu, plus particulièrement constitué par un équivalent de derme, plus particulièrement entrant dans la composition d'un équivalent de peau, notamment humaine.

L'on connaît, d'ores et déjà, des procédés permettant de fabriquer un tel équivalent de peau et consistant à prélever (usuellement par biopsie) un échantillon de peau chez un donneur, à mettre cet échantillon en culture dans des conditions appropriées, et à greffer l'équivalent de peau obtenu par cette culture.

Un tel procédé présente, cependant, un certain nombre d'inconvénients.

En particulier, dans un bref délai après le prélèvement (notamment immédiatement après la biopsie) et/ou au cours du temps de culture, l'échantillon de peau prélevé ou l'équivalent de peau a tendance à se rétracter.

Cette rétraction s'accompagne, pour l'équivalent de peau, d'une modification des caractéristiques fonctionnelles initiales que présentent les cellules de la peau à prélever.

Finalement, la rétraction de l'équivalent de peau greffé s'accompagne d'une modification particulièrement inesthétique de la surface de la peau, ceci au niveau du greffon, voire encore en périphérie de celui-ci.

La présente invention se veut à même de remédier aux inconvénients des procédés de culture de derme et de peau de l'état de la technique.

A cet effet, l'invention concerne un procédé pour la réalisation d'un équivalent de peau, notamment humaine, comportant au moins un équivalent de derme ainsi qu'au moins un équivalent d'épiderme.

Ce procédé est caractérisé par le fait qu'il consiste en ce que :
- on réalise, tout d'abord, un équivalent tissulaire non rétracté (T) constitué par un équivalent de derme de peau (D) en mettant en oeuvre un procédé consistant en ce que :
   - on réalise un mélange contenant au moins un élément entrant dans la composition d'une matrice extracellulaire ;
   - on imbibe, avec une partie au moins de ce mélange, au moins un support, au moins en partie constitué par une structure pluridimensionnelle comportant des cavités sous forme d'interstices et/ou d'alvéoles, réalisé en un matériau biocompatible, au moins en partie constitué par un matériau textile tissé et/ou tricoté, et adoptant la forme d'un cadre, ce cadre délimitant une ouverture et adoptant une forme polygonale ou ronde ;
   - à partir des composants de ce mélange, on réalise, à l'intérieur de ce support et au travers de l'ouverture de ce support, une lattice comportant au moins un élément entrant dans la composition d'une matrice extracellulaire ;
   - on fixe, sur la structure du support, au moins un élément d'une matrice extracellulaire que comporte la lattice ceci en assurant une bioadhésion, sur cette structure, d'au moins un tel élément ;
   - du fait de la fixation de la lattice sur le support, on empêche la rétraction d'au moins la lattice et on maintient au moins cette lattice sous tension sur ce support ;
   - on intègre des fibroblastes à l'intérieur de la lattice et on assure au moins une culture cellulaire de ces fibroblastes au moins dans la lattice, ceci pour l'obtention de l'équivalent tissulaire non rétracté;
- on réalise, ensuite et à la surface de l'équivalent de derme obtenu, l'équivalent d'épiderme en élaborant ou en apportant au moins une couche de kératinocytes, ceci avant d'assurer au moins une culture cellulaire de ces kératinocytes.

Une caractéristique additionnelle consiste en ce qu'on imbibe, avec une partie au moins du mélange, un support réalisé en un matériau textile, s'étendant dans au moins un plan, et présentant un maillage, délimitant les cavités, et réalisé par tissage et/ou tricotage de fibres.

Plus particulièrement, on imbibe, avec une partie au moins du mélange, un support réalisé en un matériau textile comportant au moins deux nappes textiles, s'étendant chacune dans un plan parallèle au plan d'une autre nappe textile, positionnées à une distance déterminée l'une de l'autre en sorte de définir entre elles un interstice constituant une cavité, comportant des mailles délimitant des cavités, positionnées en sorte que les mailles de deux nappes soient décalées, et raccordées entre elles par des filaments permettant d'éviter la déformation géométrique.

Cette invention concerne, encore, un équivalent de peau, notamment humaine, comportant au moins un équivalent de derme de peau ainsi qu'au moins un équivalent d'épiderme. Cet équivalent de peau est caractérisé par le fait qu'il est, notamment, obtenu par la mise en oeuvre du procédé décrit ci-dessus, et que:
- l'équivalent de derme de peau est constitué par un équivalent tissulaire non rétracté comportant :
   - un support, au moins en partie constitué par une structure pluridimensionnelle comportant des cavités sous forme d'interstices et/ou d'alvéoles, réalisé en un matériau biocompatible, au moins en partie constitué par un matériau textile tissé et/ou tricoté, et adoptant la forme d'un cadre, ce cadre délimitant une ouverture et adoptant une forme polygonale ou ronde ;

   - au moins un élément entrant dans la composition d'une matrice extracellulaire, un tel élément pénétrant à l'intérieur de la structure du support, s'étendant au travers de l'ouverture du cadre, et formant une lattice, ceci à l'intérieur de cette structure et au travers de cette ouverture ;
   - cette lattice, d'une part, comportant au moins un élément entrant dans la composition d'une matrice extracellulaire, d'autre part, fixée par bioadhésion sur la structure du support et maintenue sous tension sur ce support et, d'autre part encore, intégrant au moins des fibroblastes et/ou des myofibroblastes résultant d'une différenciation cellulaire de tels fibroblastes ;
- l'équivalent d'épiderme comporte au moins une couche de kératinocytes.

De plus, le support, réalisé en un matériau textile, s'étend dans au moins un plan, et présente un maillage, délimitant les cavités, et réalisé par tissage et/ou tricotage de fibres.

En outre, le support, réalisé en un matériau textile, comporte au moins deux nappes textiles, s'étendant chacune dans un plan parallèle au plan d'une autre nappe textile, positionnées à une distance déterminée l'une de l'autre en sorte de définir entre elles un interstice constituant une cavité, comportant des mailles délimitant des cavités, positionnées en sorte que les mailles de deux nappes soient décalées, et raccordées entre elles par des filaments permettant d'éviter la déformation géométrique.

Le procédé pour la réalisation d'un équivalent tissulaire non rétracté consiste, en particulier, à fixer, sur la structure du support, au moins un élément, entrant dans la composition d'une matrice extracellulaire, et que comporte une lattice intégrant des fibroblastes, ceci avant d'assurer au moins une culture cellulaire de ces fibroblastes.

De plus, le procédé de réalisation d'un équivalent de peau, consistant à réaliser tout d'abord un équivalent tissulaire non rétracté (constitué par un équivalent de derme de cette peau) en mettant en oeuvre le procédé susmentionné, est également mis en oeuvre alors que la lattice de l'équivalent de derme de peau est fixée sur la structure du support.

Une telle fixation permet, avantageusement et malgré la tendance naturelle à la rétraction, d'empêcher cette rétraction et de maintenir sous tension, selon le cas, l'équivalent tissulaire (plus particulièrement, l'équivalent de derme) et/ou l'équivalent de peau.

En empêchant une telle rétraction, d'une part, on améliore les qualités esthétiques de l'équivalent de peau, d'autre part, on autorise une fonctionnalisation des cellules (en particulier on autorise une différenciation myofibroblastique des fibroblastes) et, d'autre part encore, on améliore les caractéristiques fonctionnelles des cellules.

Les procédés susmentionnés consistent à réaliser, selon le cas, un équivalent tissulaire non rétracté (plus particulièrement un équivalent de derme de peau) et/ou un équivalent de peau comportant un tel équivalent de derme.

Un tel équivalent (tissulaire, de derme et/ou de peau) peut être réalisé avec une finalité de recherche, notamment pharmaceutique et/ou pharmacologique et/ou toxicologique, mais également avec une finalité thérapeutique, plus particulièrement dans le cadre de la suppléance de la fonction barrière dans les pathologies chroniques avec perte de substance cutanée (par exemple des ulcères de jambe) mais aussi dans le cadre de brûlures, escarres, plaies post-radiothérapiques ou autres.

Dans ce dernier cas et selon l'application clinique envisagée, on peut apporter soit un équivalent de derme, soit une peau de remplacement constituée par un équivalent de peau (comportant un équivalent de derme ainsi qu'un équivalent d'épiderme), ceci afin d'assurer, par greffe, le comblement d'une partie dépourvue de substance cutanée.

On observera qu'une telle greffe s'opère, là encore, en maintenant le derme équivalent et l'équivalent de peau sous tension et en empêchant leur rétraction. Ceci permet, avantageusement, d'assurer la restauration des fonctions cutanées et d'apporter les facteurs de croissance nécessaires à la cicatrisation, ceci au niveau du greffon.

Finalement, le fait de maintenir sous tension et d'empêcher la rétraction permet, avantageusement, d'augmenter de manière substantielle la surface d'équivalent tissulaire non rétracté réalisé, plus particulièrement d'équivalent de derme, et d'équivalent de peau obtenus par la mise en oeuvre du procédé selon l'invention, ceci par rapport à la surface de peau prélevée, notamment par biopsie.

Encore un autre avantage consiste en ce que le procédé conforme à l'invention permet de réaliser un équivalent de derme et un équivalent de peau destinés à être greffés sur le donneur chez lequel on aura prélevé les fibroblastes (voire encore les kératinocytes) ayant permis la réalisation d'un tel équivalent qui sera, donc avantageusement, réalisé de manière autologue et élimine tout risque de rejet du greffon.

De plus et étant donné que les cellules présentent des propriétés différentes en fonction de leur origine (bras, jambe...), le procédé conforme à l'invention permet, avantageusement, de conférer aux cellules du greffon les mêmes propriétés que celles des cellules prélevées.

Une caractéristique additionnelle consiste en ce que le procédé permet de réaliser un équivalent de peau susceptible d'être greffé directement, immédiatement et en une seule étape, contrairement à d'autres substituts de peau de l'état de la technique.

Finalement, le procédé consiste à assurer une culture cellulaire in vitro de fibroblastes qui peuvent, avantageusement, être prélevés par biopsie et dont le nombre est substantiellement inférieur à celui des greffes en pastille connues dans l'état de la technique.

D'autres buts et avantages de la présente invention apparaîtront au cours de la description qui va suivre se rapportant à des modes de réalisation qui ne sont donnés qu'à titre d'exemples indicatifs et non limitatifs.

La compréhension de cette description sera facilitée en se référant aux dessins joints en annexe et dans lesquels :
- la figure 1 est une vue schématisée d'un support destiné à être imbibé par un mélange contenant au moins un élément entrant dans la composition d'une matrice extracellulaire et au moins des fibroblastes ;
- les figures 2a, 2b, 2c, 2d sont des vues schématisées représentant les différentes étapes de la réalisation d'un équivalent tissulaire non rétracté.
- les figures 3a, 3b, 3c sont des vues schématisées représentant différentes étapes de la réalisation d'un équivalent de peau.

La présente invention concerne le domaine de la fabrication de l'équivalent d'au moins un tissu non rétracté, notamment humain, ainsi que de l'équivalent d'un organe comportant un tel équivalent tissulaire non rétracté.

A ce propos, il convient d'observer qu'un tel équivalent tissulaire non rétracté T peut être constitué (de manière non limitative) par :
- un équivalent de muqueuse ;
- un équivalent de membrane, notamment amniotique ;
- un équivalent de chorion ;
- un équivalent de tégument, plus particulièrement un équivalent de derme de peau, notamment humain.

Il est décrit, alors et dans le cadre de la fabrication d'un équivalent tissulaire non rétracté T, d'une part, un procédé pour la réalisation d'un tel équivalent tissulaire non rétracté T, notamment humain, et, d'autre part, un équivalent tissulaire non rétracté T obtenu par la mise en oeuvre de ce procédé.

Dans le cas particulier d'un équivalent tissulaire non rétracté T constitué par un équivalent de derme D de peau, notamment humain, il est décrit :
- un procédé pour la réalisation d'un équivalent tissulaire non rétracté T constitué par un équivalent de derme D ainsi qu'un tel équivalent tissulaire non rétracté T (constitué par un équivalent de derme D) obtenu par la mise en oeuvre de ce procédé ;
- un procédé pour la réalisation d'un équivalent de peau P (comportant au moins un équivalent d'épiderme E ainsi qu'au moins un équivalent tissulaire non rétracté T constitué par un équivalent de derme D) ainsi qu'un tel équivalent de peau P obtenu par la mise en oeuvre de ce procédé.

Ainsi, il est décrit, tout d'abord, un procédé pour la réalisation d'un équivalent tissulaire non rétracté T, notamment humain.

Ce procédé consiste en ce qu'on réalise un mélange m contenant au moins un élément entrant dans la composition d'une matrice extracellulaire.

Un mode particulier de réalisation consiste en ce qu'un tel mélange m est au moins en partie constitué par une telle matrice extracellulaire comportant au moins un tel élément.

En ce qui concerne ledit élément entrant dans la composition d'une matrice extracellulaire, celui-ci peut être constitué par un constituant biologique d'une telle matrice extracellulaire ou par un substitut synthétique d'un tel constituant biologique.

Un mode particulier de réalisation de l'invention consiste en ce qu'un tel élément est constitué par un constituant biologique au moins en partie défini par au moins un collagène.

A ce propos, on observera qu'un tel constituant biologique peut, alors, être défini par un unique collagène ou par une pluralité de collagènes, plus particulièrement de différents types.

Une autre caractéristique consiste en ce qu'un tel collagène présente, de préférence, un caractère acido-soluble.

Une caractéristique additionnelle consiste en ce qu'un tel élément peut se présenter, au sein du mélange m, sous forme d'une dispersion de fibres ou (et de préférence) sous forme d'une matrice extracellulaire comportant au moins un tel élément.

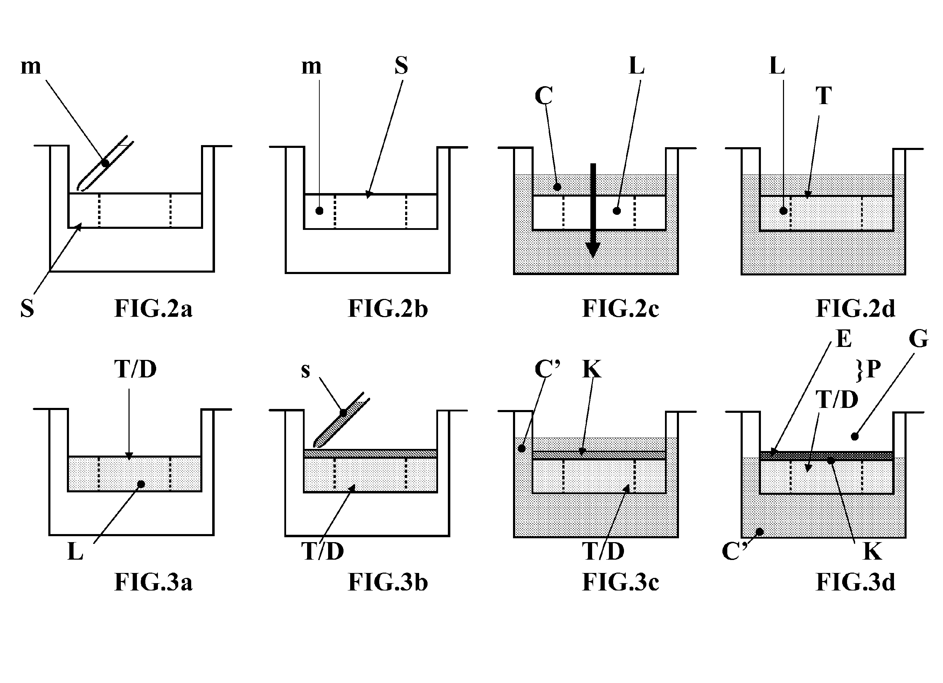

Le procédé consiste en ce que, après avoir réalisé le mélange m susmentionné, on imbibe, avec une partie au moins de ce mélange m, au moins un support S (figure 2a) présentant des caractéristiques permettant de limiter la déformabilité géométrique.

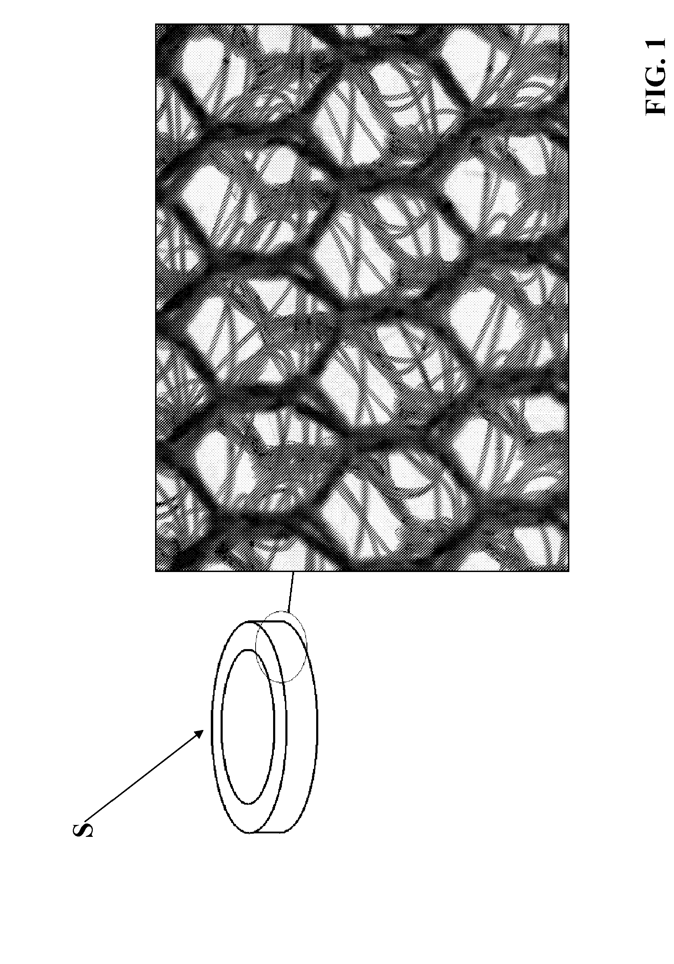

Un tel support S est au moins en partie constitué par une structure pluridimensionnelle, plus particulièrement bidimensionnelle ou (et de préférence) tridimensionnelle (figure 1).

Cette structure S pluridimensionnelle comporte des cavités se présentant sous la forme d'interstices et/ou d'alvéoles, notamment de forme hexagonale.

En fait et selon un mode préféré de réalisation, un tel support S est constitué, au moins en partie, par un matériau textile tissé et/ou tricoté, plus particulièrement à partir de fibres.

Tel que visible figure 1, un tel matériau textile présente un maillage, plus particulièrement réalisé par tissage et/ou tricotage de telles fibres, et délimitant les cavités (plus particulièrement les alvéoles) susmentionnées.

De telles alvéoles présentent, de préférence et comme visible figure 1, une forme hexagonale dont les 6 côtés ont, chacun, une longueur de l'ordre du millimètre, plus particulièrement de l'ordre de 1 mm ± 0.25 mm, de préférence de l'ordre de 1 mm ± 0.15 mm.

Une caractéristique additionnelle consiste en ce qu'un tel matériau textile s'étend dans au moins un plan. Un premier mode de réalisation, correspondant à une structure bidimensionnelle, consiste en ce qu'un tel matériau textile comporte une unique nappe textile s'étend dans un unique plan. Un deuxième mode de réalisation, correspondant à une structure tridimensionnelle et illustré figure 1, consiste en ce qu'un tel matériau textile comporte au moins deux nappes textiles :
- s'étendant chacune dans un plan parallèle au plan d'une autre nappe textile ;
- positionnées à une distance déterminée l'une de l'autre en sorte de définir entre elles un interstice constituant une cavité ;
- positionnées en sorte que les mailles, délimitant les alvéoles de ces deux nappes, soient décalées ;
- raccordées entre elles par des filaments permettant d'éviter la déformation géométrique.

Dans un tel matériau textile, les deux nappes textiles sont positionnées à une distance de l'ordre de 2 mm ± 1 mm, de préférence à une distance de l'ordre de 2 mm ± 0.5 mm.

On observera que l'utilisation d'un tel matériau textile permet de conférer à l'équivalent tissulaire (plus particulièrement à l'équivalent de derme de peau) ainsi qu'à l'équivalent de peau des propriétés optimales en termes de bioadhésion, d'épaisseur (avec, plus particulièrement, un équivalent de derme de peau dont l'épaisseur est 2 à 3 mm alors que les supports de l'état de la technique permettent l'obtention de dermes équivalents de quelques dixièmes de millimètres seulement), de prolifération cellulaire et de différenciation des fibroblastes en myofibroblastes.

Une autre caractéristique de ce support S consiste en ce qu'il est, de préférence, réalisé en un matériau biocompatible, usuellement dénommé biomatériau.

Selon une autre caractéristique, le matériau de ce support S peut encore être de type non résorbable (plus particulièrement en polyester, en polyamide -notamment en nylon- ou autre) ou, et de préférence, de type résorbable, plus particulièrement de type biorésorbable.

A ce propos, on observera que ce sont, alors, plus particulièrement les fibres du matériau textile de ce support S qui sont de type biocompatible, voire résorbables.

Une caractéristique additionnelle consiste en ce que ce support S est réalisé en un matériau présentant une élasticité et/ou une rigidité (de l'ordre de 0.050N.mm⁻¹ à 37°C en milieu aqueux) proche de celle de la peau (0.025N.mm⁻¹).

Encore une autre caractéristique consiste en ce qu'un tel support S adopte, de préférence, la forme d'un cadre délimitant une ouverture qui peut être débouchante ou traversante.

De manière additionnelle ou alternative, une telle ouverture est, de préférence, définie de manière centrale par rapport au support S.

Une autre caractéristique de ce support S consiste en ce qu'il adopte une forme polygonale (carré, rectangulaire, hexagonale) ou, et de préférence, ronde (figure 1).

Ainsi et selon un premier mode de réalisation non représenté, un tel support S peut adopter la forme d'une assiette comportant, d'une part, un tel cadre délimitant une ouverture débouchante, notamment centrale, et, d'autre part, un fond à partir duquel s'étend ce cadre, plus particulièrement en s'évasant.

Cependant et selon un mode de réalisation préféré de l'invention illustré figure 1, un tel support S adopte la forme d'un anneau, d'un tore ou analogue comportant un cadre délimitant une ouverture traversante, notamment centrale.

En fait et tel qu'il ressortira de la suite de la description, ce support S sera positionné dans un récipient (plus particulièrement une boite de Pétri ou analogue) qui adopte une forme (usuellement ronde) à laquelle la forme de ce support S sera, de préférence, adaptée.

C'est, plus particulièrement, un tel support S qui est, alors, imbibé avec une partie au moins du mélange m (figure 2b), notamment en coulant une partie au moins de ce mélange m sur ce support S disposé dans un récipient (figure 2a).

Ce procédé consiste, encore, en ce que, à partir des composants de ce mélange m, on réalise, au moins au niveau du support S, une lattice L comportant au moins un élément entrant dans la composition d'une matrice extracellulaire.

A ce propos, on observera que, à l'intérieur du mélange m contenant au moins un élément entrant dans la composition d'une matrice extracellulaire (voire contenant au moins une telle matrice extracellulaire), un tel élément (respectivement une telle matrice extracellulaire) se présente à l'état liquide ce qui permet, alors, à un tel élément (respectivement à une telle matrice extracellulaire), lorsqu'il est mis en présence dudit support S (notamment après avoir mouillé ce dernier), de pénétrer à l'intérieur de la structure de ce support S pour occuper au moins une partie (de préférence l'intégralité) du volume interne de ce support S et/ou pour se répartir de manière homogène à l'intérieur de ce support S.

Dans un pareil cas, ce sont, plus particulièrement, les fibres d'un tel élément (respectivement d'une telle matrice extracellulaire) qui vont pénétrer à l'intérieur du support S et s'enchevêtrer avec les mailles de la structure de ce support S.

Ainsi, en pénétrant à l'intérieur de la structure du support S, un tel élément (respectivement une telle matrice extracellulaire) va s'organiser pour constituer, à l'intérieur de ce support S, une lattice L.

Tel que mentionné ci-dessus, le support S adopte la forme d'un cadre présentant une ouverture. A ce propos, on observera que, de manière additionnelle à la pénétration d'un élément (respectivement d'une matrice extracellulaire) à l'intérieur de la structure du support S, une partie d'au moins un élément (respectivement d'au moins une matrice extracellulaire) que contient le mélange m va s'étendre au travers d'une telle ouverture et former, au travers de cette ouverture, une lattice **L.**

Le procédé consiste, en outre, en ce qu'on fixe, sur la structure du support S, au moins une partie des composants de ce mélange m, notamment au moins un élément que comporte la lattice L.

A ce propos, on observera que, lorsqu'on fixe au moins une partie des composants du mélange m sur la structure du support S, on assure, en fait, une bioadhésion, sur cette structure, d'au moins un élément, entrant dans la composition d'une matrice extracellulaire, et que comporte la lattice L.

Une telle bioadhésion est, plus particulièrement, réalisée par polymérisation d'au moins un tel élément de la lattice L qui s'accroche, alors, aux mailles de la structure du support S.

De manière avantageuse, une telle bioadhésion est facilitée par l'utilisation d'un support S présentant les caractéristiques susmentionnées.

Le procédé de réalisation d'un équivalent tissulaire non rétracté T consiste, alors, en ce qu'on empêche la rétraction d'au moins la lattice L et en ce qu'on maintient au moins cette lattice L sous tension sur le support S.

A ce propos, on observera qu'un tel maintien sous tension résulte, d'une part, de la fixation de cette lattice L sur le support S et, d'autre part, de la présence, au sein de la lattice L, de forces de contraction qui auraient tendance a provoquer la rétraction de l'équivalent tissulaire T mais qui, du fait de la fixation de la lattice L sur le support S, empêche cette rétraction et permet à l'équivalent tissulaire T de rester isométrique.

De manière avantageuse, un tel maintien sous tension permet d'optimiser l'organisation de la lattice L, notamment par rapport au support S.

Une caractéristique additionnelle du procédé consiste en ce qu'on intègre des fibroblastes à l'intérieur de la lattice L.

Selon un premier mode de réalisation, de tels fibroblastes peuvent être incorporés à l'intérieur de la lattice L en réalisant, tout d'abord, un mélange m contenant de tels fibroblastes, ceci avant de réaliser une telle lattice L à partir d'un tel mélange m et conformément au procédé susmentionné.

Cependant et selon un autre mode de réalisation, on intègre de tels fibroblastes en réalisant, tout d'abord, la lattice L tel que mentionné ci-dessus et à partir d'un mélange m dépourvu de fibroblastes, ceci avant de faire migrer de tels fibroblastes à l'intérieur de cette lattice L.

A ce propos, on observera que de tels fibroblastes peuvent être de différentes provenances.

En particulier, de tels fibroblastes peuvent être prélevés sur un donneur, plus particulièrement celui auquel est destiné l'équivalent tissulaire T (plus particulièrement l'équivalent de derme D) obtenu par le procédé décrit ci-dessus et chez lequel sera greffé l'équivalent tissulaire T (greffe autologue) réalisé par ce procédé.

Le prélèvement de ces fibroblastes peut, notamment, être assuré par biopsie de la peau du donneur.

Une autre caractéristique du procédé consiste en ce qu'on assure au moins une culture cellulaire des fibroblastes au moins dans la lattice L, ceci pour l'obtention de l'équivalent tissulaire non rétracté T.

A ce propos, on observera qu'une telle culture cellulaire des fibroblastes est assurée en immergeant, dans un milieu de culture C, au moins une partie (voire et de préférence l'ensemble) de la lattice L contenant au moins des fibroblastes (figure 2c).

Pour ce faire et selon un premier mode de réalisation, on immerge le support S dans un tel milieu de culture C avant de fixer au moins une partie des composants du mélange m susmentionné sur la structure de ce support S.

Cependant et selon un mode de réalisation préféré de l'invention, on immerge ce support S dans un tel milieu de culture C après avoir fixé au moins une partie des constituants de ce mélange m sur la structure de ce support S.

En ce qui concerne ce milieu de culture C, celui-ci est constitué par un milieu de type DMEM qui peut contenir (ou non) un sérum (plus particulièrement un sérum de type Sérum de Veau Foetal) et/ou au moins un antibiotique.

Une autre caractéristique du procédé consiste en ce que l'immersion d'au moins une partie de la lattice L est assurée pendant une durée comprise entre 3 et 7 jours, de préférence de l'ordre de 5 jours.

Il convient d'observer que la culture cellulaire des fibroblastes est, avantageusement, optimisée par le maintien sous tension de la lattice L sur la structure du support S et/ou par l'utilisation d'un support S présentant les caractéristiques susmentionnées.

En fait et conformément au procédé, lorsqu'on assure une culture cellulaire des fibroblastes, on réalise, dans le milieu de culture C, une prolifération et/ou une différenciation de ces fibroblastes, ceci au moins au sein de la lattice L (figure 2d) et pour l'obtention de l'équivalent tissulaire non rétracté T.

De manière avantageuse, la prolifération des fibroblastes est accentuée par l'utilisation d'un support S présentant les caractéristiques susmentionnées.

En ce qui concerne la différenciation des fibroblastes, celle-ci consiste, plus particulièrement, en une maturation et/ou en une fonctionnalisation cellulaire de fibroblastes en myofibroblastes qui expriment l'α-sm actine et qui possèdent des propriétés contractiles.

Une telle différenciation cellulaire des fibroblastes (plus particulièrement une maturation/fonctionnalisation de ces fibroblastes) est, avantageusement et là encore, optimisée par le maintien sous tension de la lattice L sur la structure du support S et/ou par l'utilisation d'un support S présentant les caractéristiques susmentionnées.

De même l'utilisation d'un tel support S permet d'accroître notablement l'épaisseur de l'équivalent tissulaire T réalisé par la mise en oeuvre de ce procédé (de l'ordre de 2 à 3mm), ceci par rapport aux techniques de l'art antérieur qui permettent l'obtention d'un équivalent tissulaire T dont l'épaisseur est notablement moindre.

En mettant en oeuvre le procédé décrit ci-dessus, on réalise un équivalent tissulaire T (notamment un équivalent de derme D de peau) qui présente, de manière avantageuse, une structure comparable ainsi que des propriétés (notamment en terme de caractéristiques mécaniques, d'épaisseur, de fonctionnalité cellulaire...) proches de celles d'un tissu naturel (notamment de derme de peau naturel) in situ. Un tel équivalent tissulaire T pourra, alors, avantageusement, être utilisé comme modèle cutané pour des essais à finalité de recherche (notamment pharmacotoxicologiques), mais également avec une finalité thérapeutique.

Encore une autre caractéristique consiste en ce que le procédé permet de réaliser un équivalent tissulaire non rétracté T de manière autologue ou, encore, de manière allogénique ou xénogénique.

Tel que mentionné ci-dessus, le procédé permet de réaliser un équivalent tissulaire non rétracté T qui est constitué (à titre d'exemple et de manière aucunement limitative) par:
- un équivalent de muqueuse ;
- un équivalent de membrane, notamment amniotique ;
- un équivalent de chorion ;
- un équivalent de tégument, plus particulièrement un équivalent de derme de peau D, notamment humain.

La présente invention concerne un procédé pour la réalisation d'un équivalent de peau P, notamment humaine, comportant au moins un équivalent de derme D ainsi qu'au moins un équivalent d'épiderme E.

Selon l'invention, ce procédé consiste en ce qu'on réalise, tout d'abord, un équivalent tissulaire non rétracté T constitué par un équivalent de derme de peau D, ceci en mettant en oeuvre le procédé de réalisation d'un tel équivalent tissulaire T décrit ci-dessus.

Ce procédé de réalisation d'équivalent de peau P consiste, ensuite, en ce qu'on réalise, à la surface de l'équivalent de derme D obtenu (figure 3a), un équivalent d'épiderme E en élaborant ou en apportant au moins une couche K de kératinocytes, ceci avant d'assurer au moins une culture cellulaire de ces kératinocytes.

En ce qui concerne de tels kératinocytes, ceux-ci peuvent être obtenus par tout moyen mais sont, de préférence, prélevés (notamment par biopsie) sur un donneur, plus particulièrement celui auquel est destiné l'équivalent de peau P obtenu par le procédé conforme à l'invention et chez lequel sera greffé cet équivalent de peau P (greffe autologue) réalisé par ce procédé.

Tel que mentionné ci-dessus, le procédé consiste en ce qu'on réalise un équivalent d'épiderme E en élaborant au moins une couche K de kératinocytes.

Aussi et conformément à l'invention, lorsqu'on élabore au moins une couche K de kératinocytes, on ensemence la surface de l'équivalent de derme D en déposant, sur cette surface, une suspension s contenant des kératinocytes (figure 3b) et on assure au moins une culture cellulaire des kératinocytes ensemencés.

En fait, la surface de l'équivalent de derme D est ensemencée avec entre 10⁵ et 10⁶ kératinocytes par cm².

Après avoir ensemencé la surface de l'équivalent de derme D avec des kératinocytes, le procédé consiste en ce qu'on assure une culture cellulaire des kératinocytes, ceci pour l'obtention d'au moins une couche K de kératinocytes.

Une telle culture cellulaire peut, également, être assurée après avoir apporté au moins une couche K de kératinocytes à la surface de l'équivalent de derme D.

A ce propos, on observera qu'une telle culture cellulaire est assurée en immergeant (figure 3c), dans un milieu de culture C' (qui peut ou non présenter, sensiblement, les mêmes caractéristiques que le milieu de culture C susmentionné), au moins l'équivalent de derme D, voire encore les kératinocytes ensemencés ou apportés à la surface de cet équivalent de derme D.

En fait, une telle immersion est assurée pendant une durée pouvant aller jusqu'à 7 jours.

Après l'obtention d'une monocouche K de kératinocytes (constituant, en fait déjà, un équivalent d'épiderme E), le procédé de réalisation d'un équivalent de peau P peut être interrompu, notamment pour une utilisation d'un tel équivalent de peau P avec une finalité de recherche (notamment pharmaceutique, pharmacologique ou toxicologique).

Cependant et selon un mode préféré de réalisation, ce procédé peut être poursuivi en sorte que, après élaboration ou apport d'une telle monocouche K de kératinocytes, on induit une différenciation cellulaire des kératinocytes de cette couche K, ceci pour l'obtention d'un équivalent d'épiderme E pluristratifié.

A ce propos, il convient d'observer qu'on induit une telle différenciation cellulaire en émergeant, pendant une durée déterminée, au moins une partie de la couche K de kératinocytes, ceci en dehors du milieu de culture C' et à l'interface entre ce milieu de culture C' et un environnement gazeux G.

En fait et selon un mode de réalisation préféré illustré figure 3d, on induit une telle différenciation cellulaire en émergeant l'intégralité de cette couche K de kératinocytes, ceci en dehors du milieu de culture C' et à l'interface entre ce milieu de culture C' et un environnement gazeux G.

En ce qui concerne la durée pendant laquelle au moins une partie de la couche K de kératinocytes est émergée, celle-ci peut aller jusqu'à 20 jours.

En ce qui concerne l'environnement gazeux, celui-ci est au moins en partie constitué par de l'air (plus particulièrement de l'air ambiant), notamment complété par un additif.

A ce propos, on observera qu'un tel additif peut, alors, être constitué par du dioxyde de carbone.

En mettant en oeuvre le procédé décrit ci-dessus, on réalise un équivalent de peau P qui présente, de manière avantageuse, une structure comparable ainsi que des propriétés (notamment en terme de caractéristiques mécaniques, d'épaisseur, de fonctionnalité cellulaire...) proches de celles de la peau naturelle in situ.

Ceci est essentiellement dû au fait que l'équivalent de derme D (plus particulièrement la lattice L que comporte cet équivalent de derme D) obtenu par la mise en oeuvre du procédé décrit ci-dessus est beaucoup mieux organisé qu'un équivalent de derme D réalisé en mettant en oeuvre un procédé de l'état de la technique.

En fait, cette meilleure organisation résulte, de manière essentielle, du maintien sous tension de ladite lattice L sur le support S en empêchant ainsi sa rétraction naturelle.

L'équivalent de peau P (plus particulièrement un équivalent de derme D épidermisé) obtenu par la mise en oeuvre de ce procédé pourra, avantageusement, être utilisé avec une finalité de recherche, notamment pharmaceutique et/ou pharmacologique, notamment comme modèle cutané pour des essais pharmacologiques et/ou toxicologiques.

Cependant, cet équivalent de peau P peut, également, être utilisé à des fins thérapeutiques, notamment comme greffon dans le cadre d'une greffe (notamment de type autologue) d'un tel équivalent de peau P.

Ainsi, il a été décrit ci-dessus un procédé pour la réalisation d'un équivalent tissulaire non rétracté T (notamment un équivalent de derme D de peau) ainsi qu'un procédé pour la réalisation d'un équivalent de peau P.

Il est, alors également, décrit un équivalent tissulaire non rétracté T (notamment humain), plus particulièrement (mais pas exclusivement) obtenu par la mise en oeuvre du procédé décrit ci-dessus.

Tel que mentionné ci-dessus, un tel équivalent tissulaire non rétracté T peut, alors et à titre d'exemple non limitatif, être constitué par :
- un équivalent de muqueuse ;
- un équivalent de membrane, notamment amniotique ;
- un équivalent de chorion ;
- un équivalent de tégument, plus particulièrement un équivalent de derme de peau D, notamment humain.

Un tel équivalent tissulaire T comporte, alors :
- un support S (plus particulièrement présentant les caractéristiques décrites ci-dessus) au moins en partie constitué par une structure pluridimensionnelle comportant des cavités ;
- une lattice L, d'une part, comportant au moins un élément (plus particulièrement présentant les caractéristiques décrites ci-dessus) entrant dans la composition d'une matrice extracellulaire, d'autre part, fixée (plus particulièrement comme décrit ci-dessus, notamment par bioadhésion) et maintenue sous tension sur ce support S et, d'autre part encore, intégrant au moins des fibroblastes et/ou des myofibroblastes résultant d'une différenciation cellulaire de tels fibroblastes.

L'invention concerne, également, un équivalent de peau P (notamment humaine) comportant, d'une part, au moins un équivalent de derme D et, d'autre part, au moins un équivalent d'épiderme E.

En fait, un tel équivalent de peau P peut, notamment (mais pas exclusivement), être (au moins en partie) obtenu par la mise en oeuvre du procédé décrit ci-dessus.

En particulier, au moins l'équivalent de derme D de cet équivalent de peau P peut être obtenu par la mise en oeuvre du procédé correspondant et décrit ci-dessus et/ou présenter les caractéristiques décrites ci-dessus.

Cet équivalent de derme D de peau peut, alors et de préférence, être constitué par un équivalent tissulaire non rétracté T présentant les caractéristiques décrites ci-dessus et/ou être obtenu par la mise en oeuvre du procédé correspondant et décrit ci-dessus.

De plus, cet équivalent de peau P présente les caractéristiques décrites ci-dessus.

En particulier, cet équivalent de peau P comporte un équivalent de derme D ainsi qu'un équivalent d'épiderme E présentant, chacun, les caractéristiques décrites ci-dessus.

Ainsi, l'équivalent d'épiderme E comporte au moins une couche K de kératinocytes.

Cependant et selon une autre caractéristique de cet équivalent de peau P, l'équivalent d'épiderme E comporte, en fait, une pluralité de strates superposées de kératinocytes pour l'obtention d'un équivalent d'épiderme pluristratifié.

A ce propos, on observera que chaque strate contient des kératinocytes présentant un état de différenciation différent de celui d'une autre strate.

Finalement, l'invention concerne, encore, un support S présentant les caractéristiques mentionnées ci-dessus ainsi que l'utilisation d'un tel support S pour la réalisation, selon le cas, d'un équivalent de derme D ou d'un équivalent de peau P (notamment humaine).

## Revendications

1. Procédé pour la réalisation d'un équivalent de peau (P), notamment humaine, comportant au moins un équivalent de derme (D) ainsi qu'au moins un équivalent d'épiderme (E), ce procédé est **caractérisé par le fait qu'**il consiste en ce que :
- on réalise, tout d'abord, un équivalent tissulaire non rétracté (T) constitué par un équivalent de derme de peau (D) en mettant en oeuvre un procédé consistant en ce que :
- on réalise un mélange (m) contenant au moins un élément entrant dans la composition d'une matrice extracellulaire ;
- on imbibe, avec une partie au moins de ce mélange (m), au moins un support (S), au moins en partie constitué par une structure pluridimensionnelle comportant des cavités sous forme d'interstices et/ou d'alvéoles, réalisé en un matériau biocompatible, au moins en partie constitué par un matériau textile tissé et/ou tricoté, et adoptant la forme d'un cadre, ce cadre délimitant une ouverture et adoptant une forme polygonale ou ronde ;
- à partir des composants de ce mélange (m), on réalise, à l'intérieur de ce support (S) et au travers de l'ouverture de ce support (S), une lattice (L) comportant au moins un élément entrant dans la composition d'une matrice extracellulaire ;
- on fixe, sur la structure du support (S), au moins un élément d'une matrice extracellulaire que comporte la lattice (L), ceci en assurant une bioadhésion, sur cette structure, d'au moins un tel élément ;
- du fait de la fixation de la lattice (L) sur le support (S), on empêche la rétraction d'au moins la lattice (L) et on maintient au moins cette lattice (L) sous tension sur ce support (S) ;
- on intègre des fibroblastes à l'intérieur de la lattice (L) et on assure au moins une culture cellulaire de ces fibroblastes au moins dans la lattice (L), ceci pour l'obtention de l'équivalent tissulaire non rétracté (T) ;
- on réalise, ensuite et à la surface de l'équivalent de derme (D) obtenu, l'équivalent d'épiderme (E) en élaborant ou en apportant au moins une couche (K) de kératinocytes, ceci avant d'assurer au moins une culture cellulaire de ces kératinocytes.

2. Procédé pour la réalisation d'un équivalent de peau (P) selon la revendication 1, **caractérisé par le fait que** le mélange (m), soit contient au moins un élément entrant dans la composition d'une matrice extracellulaire, cet élément étant constitué par un constituant biologique d'une telle matrice extracellulaire, notamment un collagène à caractère acido-soluble, ou par un substitut synthétique d'un tel constituant biologique, soit est au moins en partie constitué par une telle matrice extracellulaire comportant au moins un tel élément.

3. Procédé pour la réalisation d'un équivalent de peau (P) selon l'une quelconque des revendications précédentes, **caractérisé par le fait qu'**on imbibe, avec une partie au moins du mélange (m), un support (S) réalisé en un matériau textile, s'étendant dans au moins un plan, et présentant un maillage, délimitant les cavités, et réalisé par tissage et/ou tricotage de fibres.

4. Procédé pour la réalisation d'un équivalent de peau (P) selon l'une quelconque des revendications précédentes, **caractérisé par le fait qu'**on imbibe, avec une partie au moins du mélange (m), un support (S) réalisé en un matériau textile comportant au moins deux nappes textiles, s'étendant chacune dans un plan parallèle au plan d'une autre nappe textile, positionnées à une distance déterminée l'une de l'autre en sorte de définir entre elles un interstice constituant une cavité, comportant des mailles délimitant des cavités, positionnées en sorte que les mailles de deux nappes soient décalées, et raccordées entre elles par des filaments permettant d'éviter la déformation géométrique.

5. Procédé pour la réalisation d'un équivalent de peau (P) selon l'une quelconque des revendications précédentes, **caractérisé par le fait qu'**on imbibe, avec une partie au moins du mélange (m), un support (S) réalisé en un matériau résorbable, plus particulièrement biorésorbable.

6. Procédé pour la réalisation d'un équivalent de peau (P) selon l'une quelconque des revendications précédentes, **caractérisé par le fait qu'**on assure une culture cellulaire des fibroblastes en immergeant, dans un milieu de culture (C), au moins une partie, voire et de préférence l'ensemble, de la lattice (L) contenant au moins des fibroblastes.

7. Procédé pour la réalisation d'un équivalent de peau (P) selon l'une quelconque des revendications précédentes, **caractérisé par le fait qu'**on élabore au moins une couche (K) de kératinocytes en ensemençant la surface de l'équivalent de derme (D) en déposant, sur cette surface, une suspension (s) contenant des kératinocytes et en assurant au moins une culture cellulaire des kératinocytes ensemencés.

8. Procédé pour la réalisation d'un équivalent de peau (P) selon l'une quelconque des revendications précédentes, **caractérisé par le fait qu'**on assure une culture cellulaire des kératinocytes en immergeant, dans un milieu de culture (C'), au moins l'équivalent de derme (D).

9. Procédé pour la réalisation d'un équivalent de peau (P) selon l'une quelconque des revendications précédentes, **caractérisé par le fait que**, après élaboration ou apport d'une couche (K) de kératinocytes, on induit une différenciation cellulaire des kératinocytes de cette couche (K), en émergeant, pendant une durée déterminée, au moins une partie de la couche (K) de kératinocytes, ceci en dehors du milieu de culture (C') et à l'interface entre ce milieu de culture (C') et un environnement gazeux (G).

10. Equivalent de peau (P), notamment humaine, comportant au moins un équivalent de derme de peau (D) ainsi qu'au moins un équivalent d'épiderme (E), **caractérisé par le fait que** cet équivalent de peau (P) est, notamment, obtenu par la mise en oeuvre du procédé selon l'une quelconque des revendications 1 à 9, et que:
- l'équivalent de derme de peau (D) est constitué par un équivalent tissulaire non rétracté (T) comportant :
- un support (S), au moins en partie constitué par une structure pluridimensionnelle comportant des cavités sous forme d'interstices et/ou d'alvéoles, réalisé en un matériau biocompatible, au moins en partie constitué par un matériau textile tissé et/ou tricoté, et adoptant la forme d'un cadre, ce cadre délimitant une ouverture et adoptant une forme polygonale ou ronde ;
- au moins un élément entrant dans la composition d'une matrice extracellulaire, un tel élément pénétrant à l'intérieur de la structure du support (S), s'étendant au travers de l'ouverture du cadre, et formant une lattice (L), ceci à l'intérieur de cette structure (S) et au travers de cette ouverture ;
- cette lattice (L), d'une part, comportant au moins un tel élément entrant dans la composition d'une matrice extracellulaire, d'autre part, étant fixée par bioadhésion sur la structure du support (S) et maintenue sous tension sur ce support (S) et, d'autre part encore, intégrant au moins des fibroblastes et/ou des myofibroblastes résultant d'une différenciation cellulaire de tels fibroblastes.
- l'équivalent d'épiderme (E) comporte au moins une couche (K) de kératinocytes.

11. Equivalent de peau (P) selon la revendication 10, **caractérisé par le fait que** le support (S) est réalisé en un matériau textile, s'étend dans au moins un plan, et présente un maillage, délimitant les cavités, et réalisé par tissage et/ou tricotage de fibres.

12. Equivalent de peau (P) selon l'une quelconque des revendications 10 ou 11, **caractérisé par le fait que** le support (S) est réalisé en un matériau textile comportant au moins deux nappes textiles, s'étendant chacune dans un plan parallèle au plan d'une autre nappe textile, positionnées à une distance déterminée l'une de l'autre en sorte de définir entre elles un interstice constituant une cavité, comportant des mailles délimitant des cavités, positionnées en sorte que les mailles de deux nappes soient décalées, et raccordées entre elles par des filaments permettant d'éviter la déformation géométrique.

13. Equivalent de peau (P) selon l'une quelconque des revendications 10 à 12, **caractérisé par le fait que** le support (S) est réalisé en un matériau résorbable, plus particulièrement biorésorbable.

14. Equivalent de peau (P) selon l'une quelconque des revendications 10 à 13, **caractérisé par le fait que** la lattice (L) est fixée par bioadhésion sur la structure du support (S), ceci par polymérisation d'au moins un élément, que comporte cette lattice (L), et qui entre dans la composition de la matrice extracellulaire.

15. Equivalent de peau (P) selon l'une quelconque des revendications 10 à 14, **caractérisé par le fait que** l'élément, entrant dans la composition d'une matrice extracellulaire, est, soit un constituant biologique d'une telle matrice extracellulaire, notamment au moins en partie défini par un collagène à caractère acido-soluble, soit un substitut synthétique d'un tel constituant biologique.

16. Equivalent de peau (P) selon l'une quelconque des revendications 10 à 15, **caractérisé par le fait que** l'équivalent d'épiderme (E) comporte une pluralité de strates superposées de kératinocytes, chaque strate contenant des kératinocytes présentant un état de différenciation différent de celui d'une autre strate.

## Patentansprüche

1. Verfahren zur Herstellung eines Hautäquivalents (P), insbesondere eines menschlichen Hautäquivalents, umfassend mindestens ein Dermisäquivalent (D) und mindestens ein Epidermisäquivalent (E), dieses Verfahren ist **dadurch gekennzeichnet, dass** es darin besteht, dass :
- zunächst ein ungeschrumpftes Gewebeäquivalent (T), bestehend aus einem Hautdermisäquivalent (D), hergestellt wird, indem ein Verfahren durchgeführt wird, das darin besteht, dass :
- eine Mischung hergestellt wird (m), die mindestens ein Element enthält, das in die Zusammensetzung einer extrazellulären Matrix eingeht;
- mindestens ein Träger (S), der zumindest teilweise aus einer mehrdimensionalen Struktur besteht, die als Spalte und/oder Mulden ausgestaltete Hohlräume umfasst, der aus einem biokompatiblen Material hergestellt ist, der zumindest teilweise aus einem gewebten und/oder gestrickten Textilmaterial besteht und die Form eines Rahmens annimmt, mit mindestens einem Teil dieser Mischung (M) getränkt wird, wobei dieser Rahmen eine Öffnung abgrenzt und eine polygonale oder runde Form annimmt;
- aus den Komponenten der Mischung (m) in diesem Träger (S) und durch die Öffnung dieses Trägers (S) hindurch ein Gitter (L) hergestellt wird, das mindestens ein in die Zusammensetzung einer extrazellulären Matrix eingehendes Element umfasst;
- auf der Struktur des Trägers (S) mindestens ein Element einer extrazellulären Matrix, welche das Gitter (L) umfasst, befestigt wird, und zwar dadurch, dass eine Bioadhäsion auf dieser Struktur von zumindest einem derartigen Element sichergestellt wird ;
- zufolge der Befestigung des Gitters (L) auf dem Träger (S) wird die Einschrumpfung von zumindest dem Gitter (L) verhindert und wird zumindest dieses Gitter (L) auf diesem Träger (S) gespannt gehalten ;
- es werden Fibroblasten in das Gitter (L) integriert und es wird mindestens eine Zellkultur dieser Fibroblasten zumindest in dem Gitter (L) gesichert, und zwar um ein ungeschrumpftes Gewebeäquivalent (T) zu erhalten ;
- dann wird auf der Oberfläche des erhaltenen Dermisäquivalents (D) das Epidermisäquivalent (E) hergestellt, indem zumindest eine Schicht (K) von Keratinozyten gebildet oder bereitgestellt wird, und zwar bevor zumindest eine Zellkultur dieser Keratinozyten sichergestellt wird.

2. Verfahren zur Herstellung eines Hautäquivalents (P) nach Anspruch 1, **dadurch gekennzeichnet, dass** die Mischung (m) entweder mindestens ein in die Zusammensetzung einer extrazellulären Matrix eingehendes Element enthält, wobei dieses Element aus einem biologischen Bestandteil einer solchen extrazellulären Matrix, nämlich einem Kollagen säurelöslicher Art, oder aus einen synthetischen Ersatz eines solchen biologischen Bestandteils besteht, oder zumindest teilweise aus einer solchen extrazellulären Matrix, die mindestens ein solches Element umfasst, besteht.

3. Verfahren zur Herstellung eines Hautäquivalents (P) nach irgendeinem der vorhergehenden Ansprüche, **dadurch gekennzeichnet, dass** ein aus einem Textilmaterial hergestellten Träger (S), der sich in zumindest einer Ebene erstreckt und eine Masche aufweist, die Hohlräume abgrenzt, und der durch Weben und/oder Stricken von Fasern hergestellt ist, mit zumindest einem Teil des Gemisches (m) getränkt wird.

4. Verfahren zur Herstellung eines Hautäquivalents (P) nach irgendeinem der vorhergehenden Ansprüche, **dadurch gekennzeichnet, dass** ein Träger (S), der aus einem Textilmaterial hergestellt ist, das mindestens zwei Textilbahnen umfasst, die sich jeweils in einer Ebene parallel zu der Ebene eines anderen Textilbahn erstrecken, die in einem bestimmten Abstand voneinander positioniert sind, so dass zwischen ihnen einen Spalt definiert wird, der einen Hohlraum definiert, die Hohlräume abgrenzende Maschen umfassen, die so positioniert, dass die Maschen von zwei Bahnen zu einander versetzt und miteinander durch Fäden verbunden sind, die es erlauben, die geometrische Verformung zu vermeiden, mit zumindest einem Teil des Gemisches (m) getränkt wird.

5. Verfahren zur Herstellung eines Hautäquivalents (P) nach irgendeinem der vorhergehenden Ansprüche, **dadurch gekennzeichnet, dass** ein Träger (S), der aus einem absorbierbaren, insbesondere bio-absorbierbaren Material hergestellt ist, mit zumindest einem Teil des Gemisches (m) getränkt wird.

6. Verfahren zur Herstellung eines Hautäquivalents (P) nach irgendeinem der vorhergehenden Ansprüche, **dadurch gekennzeichnet, dass** eine Zellkultur der Fibroblasten gesichert wird, indem mindestens ein Teil, sogar und vorzugsweise die Gesamtheit, des Gitters (L), das mindestens Fibroblasten enthält, in ein Kulturmedium (C) eingetaucht wird.

7. Verfahren zur Herstellung eines Hautäquivalents (P) nach irgendeinem der vorhergehenden Ansprüche, **dadurch gekennzeichnet, dass** eine Schicht (K) von Keratinozyten gebildet wird, indem die Oberfläche des Dermisäquivalents (D) dadurch ausgesät wird, dass auf dieser Oberfläche eine Suspension (s), die Keratinozyten enthält, aufgebracht wird und mindestens eine Zellkultur der ausgesäten Keratinocyten gesichert wird.

8. Verfahren zur Herstellung eines Hautäquivalents (P) nach irgendeinem der vorhergehenden Ansprüche, **dadurch gekennzeichnet, dass** eine Zellkultur der Keratinozyten dadurch gesichert wird, dass mindestens das Dermisäquivalent (D) in einem Kulturmedium (C') eingetaucht wird.

9. Verfahren zur Herstellung eines Hautäquivalents (P) nach irgendeinem der vorhergehenden Ansprüche, **dadurch gekennzeichnet, dass** nach der Bildung oder Bereitstellung einer Schicht (K) von Keratinozyten eine Zelldifferenzierung der Keratinozyten dieser Schicht (K) dadurch induziert wird, dass während einer bestimmten Zeit mindestens ein Teil der Schicht (K) von Keratinozyten außerhalb des Kultmmediums (C') und an der Schnittstelle zwischen diesem Kulturmedium (C') und einer gasförmigen Umgebung (G) aufgetaucht wird.

10. Hautäquivalent (P), insbesondere menschliches Hautäquivalent, umfassend mindestens ein Hautdermisäquivalent (D) sowie mindestens ein Epidermisäquivalent (E), **dadurch gekennzeichnet, dass** dieses Hautäquivalent (P) nämlich durch Anwendung des Verfahrens nach irgendeinem der Ansprüche 1 bis 9 erhalten wird, und dass:
- das Hautdermisäquivalent (D) aus einem nichtgeschrumpftem Gewebeäquivalent (T) besteht, umfassend:
- einen Träger (S), der zumindest teilweise aus einer mehrdimensionalen Struktur besteht, die als Spalte und/oder Mulden gestaltete Hohlräume umfasst, der aus einem biokompatiblen Material hergestellt ist, der zumindest teilweise aus einem gewebten und/oder gestrickten Textilmaterial besteht und die Form eines Rahmens annimmt, wobei dieser Rahmen eine Öffnung abgrenzt und eine polygonale oder runde Form annimmt;
- mindestens ein in die Zusammensetzung einer extrazellulären Matrix eingehendes Element, wobei ein solches Element in die Struktur des Trägers (S) hineindringt, sich durch die Öffnung des Rahmens hindurch erstreckt und ein Gitter (L) bildet, und zwar innerhalb dieser Struktur (S) und durch diese Öffnung hindurch ;
- wobei dieses Gitter (L), einerseits, mindestens ein solches in die Zusammensetzung einer extrazellulären Matrix eingehendes Element umfasst, andererseits durch Bioadhäsion an der Struktur des Trägers (S) befestigt und auf dem Träger (S) gespannt gehalten wird, und noch andererseits, zumindest Fibroblasten und/ oder Myofibroblasten integriert, die durch eine Zelldifferenzierung solcher Fibroblasten entstehen ;
- das Epidermisäquivalent (E) umfasst zumindest eine Schicht (K) von Keratinozyten.

11. Hautäquivalent (P) nach Anspruch 10, **dadurch gekennzeichnet, dass** der Träger (S) aus einem textilen Material hergestellt ist, sich in zumindest einer Ebene erstreckt und ein Gitter aufweist, das die Hohlräume abgrenzt und durch Weben und/ oder Stricken von Fasern hergestellt ist.

12. Hautäquivalent (P) nach irgendeinem der Ansprüche 10 oder 11, **dadurch gekennzeichnet, dass** der Träger (S) aus einem textilen Material hergestellt ist, das mindestens zwei Textilbahnen umfasst, die sich jeweils in einer Ebene parallel zu der Ebene einer anderen Textilbahn erstrecken, in einem bestimmten Abstand von einander positioniert sind, so dass zwischen ihnen einen Spalt gebildet wird, der einen Hohlraum definiert, die Hohlräume abgrenzende Maschen umfassen, die so positioniert sind, dass die Maschen von zwei Bahnen zu einander versetzt und miteinander durch Fäden verbunden sind, die es erlauben, die geometrische Verformung zu vermeiden.

13. Hautäquivalent (P) nach irgendeinem der Ansprüche 10 bis 12, **dadurch gekennzeichnet, dass** der Träger (S) aus einem absorbierbaren, insbesondere bio-absorbierbaren Material hergestellt ist.

14. Hautäquivalent (P) nach irgendeinem der Ansprüche 10 bis 13, **dadurch gekennzeichnet, dass** das Gitter (L) durch Bioadhäsion an der Struktur des Trägers (S) befestigt ist, und zwar durch Polymerisation von mindestens einem Element, welches dieses Gitter (L) umfasst und das in die Zusammensetzung der extrazellulären Matrix eingeht.

15. Hautäquivalent (P) nach irgendeinem der Ansprüche 10 bis 14, **dadurch gekennzeichnet, dass** das in die Zusammensetzung einer extrazellulären Matrix eingehende Element entweder ein biologischer Bestandteil einer solchen extrazellulären Matrix, der nämlich von einem Kollagen säurelöslicher Art definiert ist, oder ein synthetischer Ersatz eines solchen biologischen Bestandteils ist.

16. Hautäquivalent (P) nach irgendeinem der Ansprüche 10 bis 15, **dadurch gekennzeichnet, dass** das Epidermisäquivalent (E) eine Vielzahl von übereinanderliegenden Schichten von Keratinozyten umfasst, wobei jede Keratinocyten enthaltende Schicht einen Differenzierungszustand aufweist, der von demjenigen einer anderen Schicht unterschiedlich ist.

## Claims

1. A method for producing a skin equivalent (P), namely a human skin equivalent, including at least one dermis equivalent (D) and at least one epidermis equivalent (E), this process being **characterized in that** it consists **in that** :
- first of all, a non-shrunken tissue equivalent (T) formed of a skin dermis equivalent (D) is produced by implementing a method consisting **in that**:
- a mixture (m) containing at least one element entering into the composition of an extracellular matrix is produced ;
- with at least part of this mixture (m) is impregnated at least one support (S), at least partly formed of a multi-dimensional structure including cavities in the form of interstices and/or alveoli, made of a biocompatible material, at least partially formed of a woven and/or knitted textile material, and adopting the form of a frame, this frame delimiting an opening and adopting a polygonal or round shape ;
- from the components of this mixture (m) is made, inside this support (S) and through the opening of this support (S), a lattice (L) including at least one element entering into the composition of an extracellular matrix ;
- onto the structure of the support (S) is fixed at least one element of an extracellular matrix the lattice (L) comprises, this while ensuring a bio-adhesion on this structure of at least one such element;
- because of the fastening of the lattice (L) to the support (S), the shrinking of at least the lattice (L) is prevented and at least this lattice (L) is maintained tensioned on this support (S) ;
- fibroblasts are integrated within the lattice (L) and at least a cell culture of these fibroblasts is ensured at least in the lattice (L), this for obtaining the non-shrunken tissue equivalent (T) ;
- then and on the surface of the dermis equivalent (D) being obtained is produced the epidermis equivalent (E) by preparing or providing at least one layer (K) of keratinocytes, this prior to ensuring at least a cell culture of these keratinocytes.

2. The method for producing a skin equivalent (P) according to claim 1, **characterized in that** the mixture (m) either contains at least one element entering into the composition of an extracellular matrix, this element being formed of a biologic constituent of such an extracellular matrix, namely a collagen of an acid-soluble nature, or of a synthetic substitute for such a biological component, or is at least partly formed of such an extracellular matrix including at least one such element.

3. The method for producing a skin equivalent (P) according to any one of the preceding claims, **characterized in that** with at least part of the mixture (m) is soaked a support (S) made of a textile material extending in at least one plane and having a mesh delimiting the cavities and made by weaving and/or knitting fibers.

4. The method for producing a skin equivalent (P) according to any one of the preceding claims, **characterized in that** with at least part of the mixture (m) is soaked a support (S) made of a textile material including at least two textile webs, each extending in a plane parallel to the plane of another textile web, positioned at a determined distance from each other so as to define between them a gap constituting a cavity, including meshes delimiting cavities, positioned so that the meshes of the two webs are offset, and connected to each other by filaments permitting to avoid the geometric deformation.

5. The method for producing a skin equivalent (P) according to any one of the preceding claims, **characterized in that** with at least part of the mixture (m) is soaked a support (S) made of an absorbable material, in particular a bio-absorbable material.

6. The method for producing a skin equivalent (P) according to any one of the preceding claims, **characterized in that** a cell culture of the fibroblasts is ensured by immersing into a culture medium (C) at least part of the, even and preferably the whole, lattice (L) containing at least fibroblasts.

7. The method for producing a skin equivalent (P) according to any one of the preceding claims, **characterized in that** at least one layer (K) of the keratinocytes is prepared by seeding the surface of the dermis equivalent (D) by depositing on said surface a suspension (s) containing keratinocytes and by ensuring at least a cell culture of the seeded keratinocytes.

8. The method for producing a skin equivalent (P) according to any one of the preceding claims, **characterized in that** a cell culture of the keratinocytes is ensured by immersing into a culture medium (C') at least the dermis equivalent (D).

9. The method for producing a skin equivalent (P) according to any one of the preceding claims, **characterized in that**, after preparing or providing a layer (K) of keratinocytes, cell differentiation of the keratinocytes of this layer (K) is induced by emersion for a given duration of at least part of the layer (K) of keratinocytes, this outside of the culture medium (C') and at the interface between this culture medium (C') and a gaseous environment (G).

10. Skin equivalent (P), in particular a human skin equivalent, including at least a skin dermis equivalent (D) and at least an epidermis equivalent (E), **characterized in that** the skin equivalent (P) is, in particular, obtained by implementing the method according to any one of claims 1 to 9, and **in that**:
- the skin dermis equivalent (D) is formed of a non-shrunken tissue equivalent (T) including:
- a support (S), at least partly formed of a multi-dimensional structure including cavities in the form of interstices and/or alveoli, made of a biocompatible material, at least partially formed of a woven and/or knitted textile material, and adopting the form of a frame, this frame delimiting an opening and adopting a polygonal or round shape;
at least one element entering into the composition of an extracellular matrix, such an element penetrating into the interior of the structure of the support (S), extending through the opening of the frame, and forming a lattice (L) this inside this structure (S) and through this opening;
this lattice (L), on the one hand, including at least one such element entering into the composition of an extracellular matrix, on the other hand, being fixed by bio-adhesion to the structure of the support (S) and held tensioned on said support (S) and, yet on the other hand, integrating at least fibroblasts and/or myofibroblasts resulting from a cell differentiation of such fibroblasts;
- the epidermis equivalent (E) includes at least one layer (K) of keratinocytes.

11. Skin equivalent (P) according to claim 10, **characterized in that** the support (S) is made of a textile material, extends in at least one plane, and has a mesh delimiting the cavities and made by weaving and/or knitting fibers.

12. Skin equivalent (P) according to any one of claims 10 or 11, **characterized in that** the support (S) is made of a textile material including at least two textile webs, each extending in a plane parallel to the plane of another textile web, positioned at a determined distance from each other so as to define between them a gap forming a cavity, comprising meshes delimiting cavities, positioned so that the meshes of two webs are offset, and connected to each other by filaments permitting to avoid the geometric distortion.

13. Skin equivalent (P) according to any one of claims 10 to 12, **characterized in that** the support (S) is made of an absorbable material, in particular a bio-absorbable material.

14. Skin equivalent (P) according to any one of claims 10 to 13, **characterized in that** the lattice (L) is fixed by bio-adhesion to the structure of the support (S), this by polymerization of at least one element that this lattice (L) includes and which enters into the composition of the extracellular matrix.

15. Skin equivalent (P) according to any one of claims 10 to 14, **characterized in that** the element entering into the composition of an extracellular matrix is either a biological component of an extracellular matrix, namely at least partially defined by a collagen of an acid-soluble nature, or a synthetic substitute for such a biological component.

16. Skin equivalent (P) according to any one of claims 10 to 15, **characterized in that** the epidermis equivalent (E) comprises a plurality of superimposed layers of keratinocytes, each layer containing keratinocytes having a state of differentiation different from that of another layer.
